# EUROPÄISCHE PATENTSCHRIFT

(11) **EP 0 758 874 B1**
(45) Veröffentlichungstag und Bekanntmachung des Hinweises auf die Patenterteilung: **22.08.2001**
(21) Anmeldenummer: 95909614.0
(22) Anmeldetag: 08.03.1995
(51) Int. Cl.: A61F 2/44, A61F 2/46

(54) **ZWISCHENWIRBEL-IMPLANTAT**
INTERVERTEBRAL IMPLANT
IMPLANT INTERVERTEBRAL

(43) Veröffentlichungstag der Anmeldung: 26.02.1997
(73) Patentinhaber: Synthes AG, Chur, 7002 Chur (CH)
(72) Erfinder: SCHLÄPFER, Johannes, Fridolin, CH-8750 Glarus (CH); HESS, Martin, CH-4434 Hölstein (CH)
(74) Vertreter: Lusuardi, Werther Giovanni, Dr.
(86) Internationale Anmeldenummer: CH9500051
(87) Internationale Veröffentlichungsnummer: WO9627348

(56) Entgegenhaltungen:
- WO-A-92/14423
- DE-U- 9 407 806
- US-A- 4 834 757
- US-A- 5 147 402

## Beschreibung

Die Erfindung bezieht sich auf ein Implantat, insbesondere für den Zwischenwirbelraum, gemäss Patentanspruch 1. Solche Implantate sind hauptsächlich dazu bestimmt, Knochenbrücken an Wirbelkörpern zu fördern und welche nach der Resektion von Diskus bzw. Zwischenwirbelscheibe zwischen Wirbelkörper und Rückgrat angebracht werden.

Es ist bekannt, dass bei Beschädigung einer Zwischenwirbelscheibe diese entfernt und der entstandene Raum mit kortikospongiösem Knochen gefüllt werden kann.

Bei dieser Methode werden die Wirbelkörper zuerst weitmöglichst mit Hilfe von Spreizer auseinandergedehnt. Eine Spezialtechnik besteht darin, dass keilförmige Elemente - sogenannte Dilatatoren - zwischen die beiden Wirbelkörper eingeführt werden, um sie schrittweise auseinander zu dehnen. Dabei wird abwechselnd links und rechts jeweils ein Dilatator mit einem 1 mm grösseren Durchmesser von posterior angebracht. Nachdem die grösstmögliche Dehnung erreicht ist, werden die Dilatatoren durch den obengenannten kortiko-spongiösen Knochen ersetzt.

Diese bekannte Technik hat den Nachteil, dass der Knochen schwierig zu handhaben und in die richtige Position zu bringen ist, wobei Korrekturen nahezu ausgeschlossen sind. Ein weiterer Nachteil dieser Technik besteht darin, dass im Zwischenwirbelraum eine rechteckige oder zylinderförmige Aussparung ausgestochen und/oder ausgefräst werden muss, um die Knochenpfropfen zwischen die ursprünglich konkaven Seiten der angrenzenden Wirbelkörper bringen zu können, was umständlich ist und zusätzlich zur Beschädigung der Wirbelkörper führt.

In der WO89/12431 ist ein hohlzylindrisches, allseitig perforiertes Implantat für den Zwischenwirbelraum offenbart, welches mit einem Schraubdeckel von der Manipulierseite her verschliessbar ist. Eine Kompressionswirkung auf allfällig in den Zylinderhohlraum eingebrachte Knochenmasse kann damit nicht ausgeübt werden und zudem würden die kleinen - nur für das Einwachsen des Knochens gedachten - Perforationen ein Auspressen von Knochenmasse verunmöglichen.

In der WO90/000037 ist ein quaderförmiges, allseitig perforiertes Implantat für den Zwischenwirbelraum offenbart, welches einen im Inneren montierten Schrauben/Keil-Mechanismus aufweist, mit welchen oben und unten je 4 Klauen ausgefahren werden können. Um präoperativ Knochenmasse in dieses Implantat einfüllen zu können, müsste zuvor der Schrauben/Keil-Mechanismus demontiert und dann wieder eingebaut werden, was sehr umständlich wäre. Aber auch in einem solchen Fall, könnte die Knochenmasse nicht aus dem Implantat austreten, weil einerseits die vorhandenen, relativ grossen Schlitze durch die ausgefahrenen Klauen verschlossen werden und anderseits die kleindimensionierten Perforationen dazu ungeeignet sind.

Die WO-A-95/08306 BECKERS bildet einen Stand der Technik nach Art. 54(3)(4) EPÜ. Grundsätzlich kann das darin beschriebene, hohl ausgebildete Implantat mit einer komprimierbaren Masse, z.B. Knochenspänen gefüllt werden, doch fehlen Mittel um die Knochenspäne derart komprimieren zu können, dass sie oben und unten aus dem Implantat herausgepresst werden könnten.

Der Erfindung liegt die Aufgabe zugrunde, ein Implantat für den Zwischenwirbelraum zuschaffen, bei welchem darin eingeführte Knochenmasse - nach erfolgter Implantation - komprimiert und gegen die angefrischten, vom Knorpel befreiten Deckplatten der anliegenden Wirbel herausgedrückt werden kann. Dadurch wird der bei einer anatomischen Fehlpassung entstandene Hohlraum mit Knochenmasse aufgefüllt.

Die Erfindung löst die gestellte Aufgabe mit einem Implantat, welches die Merkmale des Anspruchs 1 aufweist. Weitere vorteilhafte Ausgestaltungen der Erfindung sind in den abhängigen Ansprüchen gekennzeichnet.

Das in das erfindungsgemässe Implantat einführbare Kompressionselement kann durch ein Werkzeug ergriffen werden, so dass sich bei entsprechender Ausbildung des Implantates relativ mühelos eine externe Kraft und ein Moment über das Kompressionselement auf das Implantat ausüben lässt, die es ermöglicht, das Implantat zusammen mit dem Kompressionselement in den Zwischenwirbelbereich einzubringen, zu entfernen und einzeln oder zusammen zu manipulieren.

Die Vorrichtungen zur Ergreifung durch ein Werkzeug können als Ansatzpunkte derart gestaltet sein, dass eine Rotationskraft und/oder eine axiale Kraft und/oder eine seitliche Kraft auf das Kompressionselement (und über dieses auf das Implantat selbst) ausgeübt werden kann.

Bei einer vorteilhaften Ausführungsform des Kompressionselementes und des Implantates einem entsprechend konzipierten Instrument sind diese Ansatzpunkte zumindest so gestaltet, dass sie die Ausübung einer Rotationskraft auf das Kompressionselement und auf das Implantat selbst ermöglichen.

Um die Erfindung besser zu verdeutlichen, werden nachstehend einige Beispiele vorteilhafter Ausführungsformen - auf die sich die Erfindung jedoch nicht beschränkt - mit Verweisen nach den entsprechenden Zeichnungen beschrieben.

Es zeigen:
Fig. 1 eine perspektivische Darstellung des Implantates mit Kompressionselement;
Fig. 2 eine Explosionsdarstellung eines für die Manipulation des Implantates nach Fig. 1 geeigneten Instrumentes;
Fig. 3 eine perspektivische Darstellung des Instrumentes nach Fig. 2;
Fig. 4 einen teilweisen Längsschnitt durch das Instrument nach Fig. 3 im Übergangsbereich zwischen Instrument und Kompressionselement des Implantats;
Fig. 5 einen Längsschnitt durch ein im Zwischenwirbelbereich eingeführtes Implantat nach Fig. 1 vor Kompression der Knochenmasse; und
Fig. 6 einen Längsschnitt durch ein im Zwischenwirbelbereich eingeführtes Implantat nach Fig. 1 nach Kompression der Knochenmasse.

Die Erfindung und Weiterbildungen der Erfindung werden im folgenden anhand der teilweise schematischen Darstellungen mehrerer Ausführungsbeispiele noch näher erläutert.

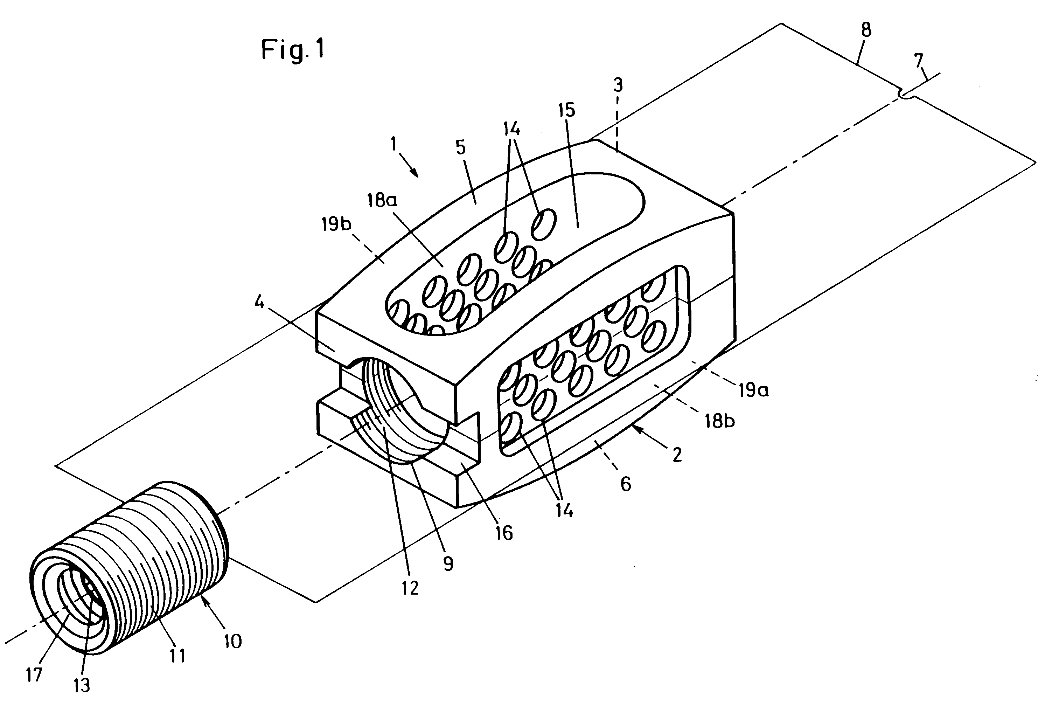

Das in Fig. 1 dargestellte Implantat 1 besteht im wesentlichen aus einem seitlich geschlossenen, oben und unten durchgehend offenen Rahmen 2 mit einer vorderen Einführungsfläche 3 und einer hinteren Manipulierfläche 4.
Die Längsachse 7 des Implantates 1 ist die Mittelsenkrechte auf die vordere Einführungsfläche 3 und hintere Manipulierfläche 4. Die Mittelebene 8 des Implantates 1 ist definiert durch die Längsachse 7 des Implantates 1 und die Mittelsenkrechte auf die seitlichen Flächen 19a,19b des Rahmens 2.

Die hintere Manipulierfläche 4 des Rahmens 2 ist mit einer Öffnung 9 versehen, in welche ein Kompressionselement 10 einführbar ist. Das Kompressionselement 10 ist vorzugsweise eine Schraube mit einem Gewinde 11 und einem Innensechskant 13. Diese Schraube wird vor dem Einführen des Implantates 1 in den Zwischenwirbelraum in die Öffnung 9 eingesetzt.

Der durch die Seitenwände 3,4,19a,19b gebildete Rahmen 2 umschliesst einen Hohlraum 15, der mit einer vorzugsweise aus autologem Knochenmaterial bestehenden komprimierbaren Masse füllbar und nach der Einführung des Implantates 1 in den Zwischenwirbelraum mittels des Kompressionselementes 10 in situ komprimierbar ist.

Die Seitenwände 19a,19b des in Fig. 1 dargestellten Implantates 1 weisen Perforationen 14 auf, durch welche der Knochen ins Implantat 1 hineinwachsen kann.

Der in der hinteren Manipulierfläche 4 befindliche Querschlitz 16 dient als Vorrichtung zur Ergreifung des Implantates 1 durch ein Instrument. Der dargestellte Querschlitz 16 ermöglich das Aufbringen eines Rotationsmomentes über das Instrument direkt auf das Implantat 1.

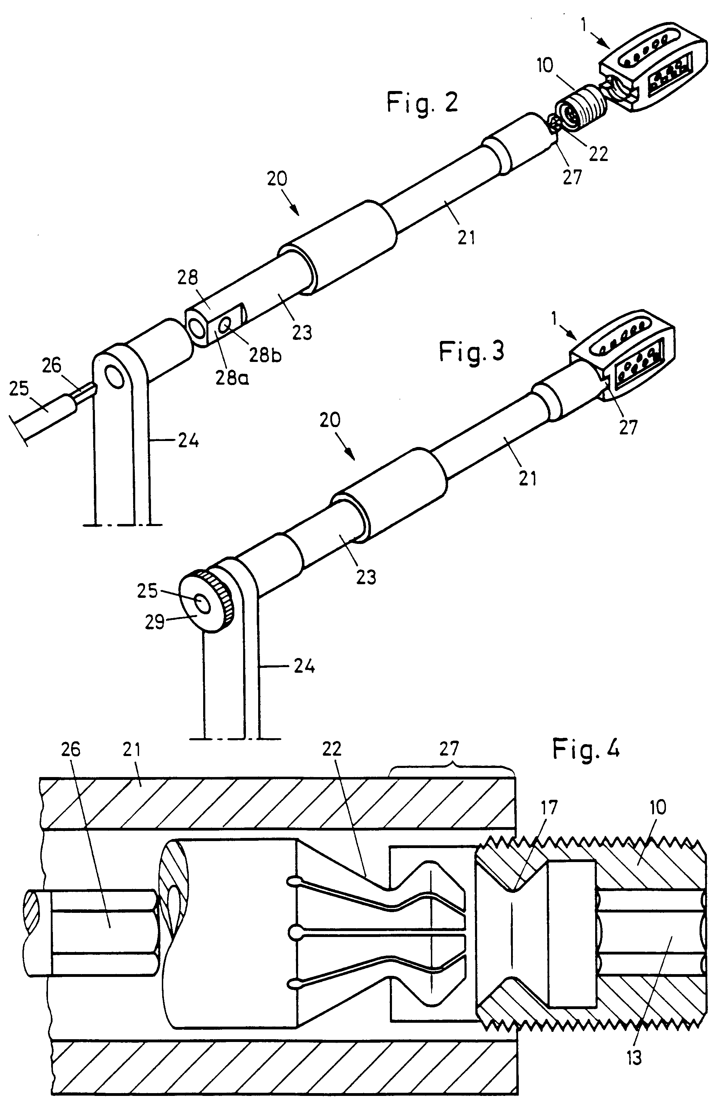

In Fig. 2 ist das Implantat 1 zusammen mit dem Kompressionselement 10 und dem Instrument 20 dargestellt. Das Instrument 20 erlaubt die gleichzeitige Manipulation des Implantates 1 und des Kompressionselementes 10. Das Instrument 20 besteht im wesentlichen aus einer Hülse 21, einem rohrartigen Einsatz 23, einem Handgriff 24 und einem weiteren Einsatz 25. Die Hülse 21 weist eine Vorrichtung 27 auf, die in die Vorrichtung 16 des Implantates 1 hineinpasst. Diese Vorrichtung 16 ermöglicht das Übertragen eines Drehmomentes vom Instrument 20 direkt auf das Implantat 1. Der Einsatz 23 wird in die Hülse 21 eingeschoben und rotationsstabil verbunden. Er weist am vorderen Ende eine Vorrichtung 22 zu einer zug- und druckfesten Verbindung mit der Kompressionsschraube 10 auf (siehe Fig. 4). Das hintere Ende 28 des Einsatzes 23 weist eine Geometrie auf, die eine rotations-, zug- und druckstabile Verbindung zwischen dem Einsatz 23 und dem Handgriff 24 ermöglicht, die Rotationsstabilität ist durch die beiden Flächen 28a gewährleistet. Zwei ko-laterale, im Handgriff 24 befindliche Kugeln werden beim Aufscheiben des Griffes 24 auf den Einsatz 23 in die ko-lateralen Bohrungen 28b hineingedrückt und erlauben somit Zug- und Druckkräfte über das Kompressionselement 10 auf das Implantat 1 anzubringen. Der zweite Einsatz wird erst eingeschoben, wenn die Vorrichtung 22 schon in das Kompressionselement 10 eingeklickt ist. Er drückt die Vorrichtung 22 auseinander und verhindert auf dies Art ein Lösen der Verbindung, wenn die Kräfte in der Längsachse des Implantates 1 auftreten. Der in Fig. 2 dargestellte zweite Einsatz 25 weist zuvorderst einen 6-Kant 26 auf, der in den 6-Kant des Kompressionselementes 10 eingeschoben wird. Dieser 6-Kant 26 erlaubt ein Ein-, bzw. Ausdrehen des als Schraube ausgebildeten Kompressionselementes 10 und ermöglicht somit ein Komprimieren der Füllmasse im Implantat 1. Der Einsatz 25 wird durch ein kleines in Fig. 3 gezeigtes Handrad 29 bedient.

In Fig. 3 ist das Instrument 20 in montiertem Zustand mit dem Einsatz 25 im Eingriff und dem montierten Handrad 29 dargestellt.

In Fig. 4 ist der untere Teil des Instrumentes 20 unmittelbar vor dem Einklicken seines spannzangenähnlichen, mit mindestens einem Schlitz versehenen Endes 23 in das Kompressionselement 10 dargestellt. Nach dem Einklicken wird der Einsatz 25 soweit in den Einsatz 23 eingeschoben bis der 6-Kant 26 vollständig in den 6-Kant 13 des Kompressionselementes 10 eingedrungen ist. In dieser Position verhindert der Einsatz 25 ein Zusammendrücken der Spannzange 22. Dank dem Flansch 17 kann nun die Verbindung zwischen Instrument 20 und Kompressionselement 10 Kräfte in der Längsrichtung des Implantates 1 aufnehmen. Wenn einmal mit dem Instrument 20 verbunden, kann das Kompressionselement 10 mit Hilfe des Einsatzes 25 gegenüber dem Instrument 20 gedreht werden. Wie schon erwähnt , ist das Instrument 20 selbst über die Vorrichtung 27 rotationsstabil mit dem Implantat 1 verbunden. Dadurch wird es nun möglich, das Kompressionselement 10 mittels des Einsatzes 25 in das Implantat 1 einzudrehen und somit die Füllmasse zu komprimieren und durch die Öffnungen 18a,18b in der oberen und unteren konvex ausgebildeten Begrenzung 5,6 des Rahmens 2 gegen die Deckplatten 130 der oben und unten angrenzenden Wirbelkörper 100 zu drücken.

Die Verbindung zwischen dem in den Fig. 2 und 3 gezeigten Instrument 20 und dem in allen Figuren dargestellten Implantat 1 wird wie folgt hergestellt:
- Einklicken der Spannzange 22 in das Kompressionselement 10
- Einschieben des Einsatzes 25 bis dessen 6-Kant 26 vollständig im 6-Kant 13 des Implantates 1 verschwunden ist;
- Einführen der Vorrichtung 27 des Instrumentes 20 in die Vorrichtung 16 des Implantates 1;
- Einführen des Kompressionselementes 10 in das Implantat 1 bis dessen vorderster Teil bündig mit der Innenseite des Rahmens 2 des Implantates 1 ist.

Jetzt kann je nach Bedarf das Implantat 1 oder das Kompressionselement 10 mit dem Instrument 20 manipuliert werden: Einführen, Rotieren und Komprimieren. Die Verbindung ist derart gestaltet, dass mittels des Instrumentes 20 vorallem Momente um und Kräfte in der Längsachse 7 auf das Kompressionselement 10 und über das Kompressionselement 10 auf das Implantat 1 ausgeübt werden können. Die vorzugsweise aus autologem Knochen bestehende Masse wird erst nach der Verbindung des Instrumentes 20 mit dem Implantat 1 und vor der Einführung des Implantates 1 in den Bandscheibenraum eingefüllt.

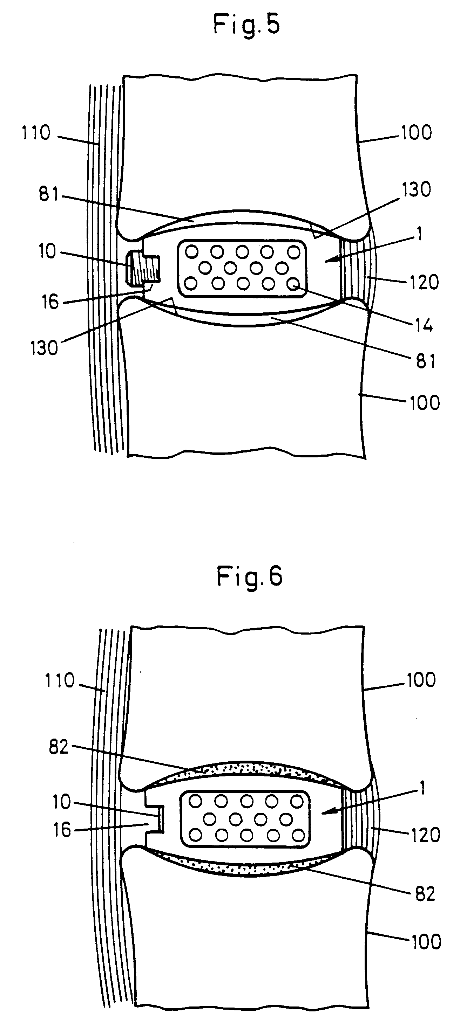

Anhand der Fig. 5 und 6 soll nun erläutert werden, wie die Füllmasse komprimiert und in den Hohlraum zwischen Implantat 1 und Knochen gepresst wird. Vorgängig werden die Implantate 1 in Querlage von posterior links und rechts an der Dura 110 vorbei mittels des Instrumentes 20 eingeführt (pro zu fusionierendes Wirbelsäulensegment zwei Implantate) und anschliessend um deren Längsachse 7 aufgerichtet werden. Nach anterior werden sie durch den restlichen Annulus 120 der Bandscheibe gehalten. Nach posterior werden sie in der Regel durch ein zusätzliches posteriores Pedikelfixationssystem stabilisiert. In den Fig. 5 und 6 ist eines dieser beiden Implantate 1 nach Einführung und Aufrichtung dargestellt. Das Kompressionselement 10 ist in der Fig. 5 noch in der Ausgangsstellung, d.h. es ist soweit eingedreht, dass das Implantat 1 über das Kompressionselement 10 mittels des Instrumentes 20 manipuliert werden kann, ohne die Füllmasse bereits zu komprimieren. Um die Übersichtlichkeit zu wahren, ist das fest mit dem Implantat 1 verbundene Instrument 20 nicht gezeichnet worden. Die in den Fig. 5 und 6 dargestellten anatomischen Strukturen zeigen eine gewisse Diskrepanz zwischen der oberen und unteren Begrenzung 5 und 6 des Implantates 1 und den Deckplatten 130 der angrenzenden Wirbelkörper 100. Das Ziel ist es nun, das Kompressionselement mittels des Einsatzes 25 soweit einzudrehen, bis die oben und unten austretende Füllmasse 82 den Hohlraum 81 zwischen Implantat 1 und Knochen aufgefüllt hat.
In Fig. 6 ist das Implantat 1 nach dem Eindrehen des Kompressionselementes 10 dargestellt. Der Hohlraum 81 ist mit der ausgetretenen Füllmasse 82 komplett gefüllt.

## Patentansprüche

1. Implantat (1), insbesondere für den Zwischenwirbelraum, bestehend aus einem, oben und unten durchgehend offenen Rahmen (2) mit einer vorderen Einführungsfläche (3) und einer hinteren Manipulierfläche (4),
wobei
A) die obere (5) und untere Begrenzung (6) des Rahmens (2) konvex ausgebildet ist, so dass das Implantat (1) in der, die Längsachse (7) des Implantats (1) enthaltenden Mittelebene (8) des Rahmens (2) insgesamt eine linsenförmige Gestalt aufweist;
B) der oben und unten durchgehend offene Innenraum (15) des Implantats (1) mit einer komprimierbaren Masse füllbar ist; und
C) die hintere Manipulierfläche (4) des Rahmens (2) eine Öffnung (9) aufweist, in welcher ein Kompressionselement (10) vorgesehen ist, welches in der Öffnung (9) unidirektional in Richtung der Längsachse (7) nach der vorderen Einführungsfläche (3) bewegbar ist, wobei
E) mittels des Kompressionselementes (10) im Innenraum (15) eingebrachte komprimierbare Masse oben und unten aus dem Implantat herauspressbar ist.

2. Implantat (1) nach Anspruch 1, dadurch gekennzeichnet, daß die Öffnung (9) mit einem Innengewinde (12) versehen ist, um ein rotationssymmetrisches Kompressionselement (10) mit entsprechendem Außengewinde (11) aufzunehmen.

3. Implantat (1) nach Anspruch 1 oder 2, dadurch gekennzeichnet, dass das Produkt P = F·T·O⁻¹ aus Querschnittsfläche F der Öffnung (9), der maximal möglichen Eindringtiefe T des Kompressionselement (10) in den Innenraum (15) und dem Reziproken der Summe der Flächen beider Öffnungen (18a,18b) in der oberen und unteren Begrenzung (5,6) des Rahmens (2) einen Wert von 0,5 bis 1,5 mm ergibt.

4. Implantat (1) nach Anspruch 3, dadurch gekennzeichnet, dass das Produkt P = F·T·O⁻¹ einen Wert von 0,6 bis 0,9 mm ergibt.

5. Implantat (1) nach Anspruch 3 oder 4, dadurch gekennzeichnet, dass das Kompressionselement (10) Mittel (13) aufweist, vorzugsweise in Form eines Innensechskantes, über welche ein Rotationsmoment ausgeübt werden kann.

6. Implantat (1) nach einem der Ansprüche 3 - 5, dadurch gekennzeichnet, dass das Kompressionselement (10) Mittel (17), vorzugsweise in Form einer Bohrung mit Absatz, aufweist, über die Kräfte in axialer Richtung ausübbar sind.

7. Implantat (1) nach einem der Ansprüche 1 - 6, dadurch gekennzeichnet, dass die hintere Manipulierfläche (4) Mittel (16) aufweist, vorzugsweise einen Querschlitz, über welche ein Rotationsmoment ausübbar ist.

8. Implantat (1) nach einem der Ansprüche 3 - 5, dadurch gekennzeichnet, dass das Implantat (1) und das Kompressionselement (10) Mittel (16) aufweisen - vorzugsweise in Form eines Querschlitzes in der hinteren Manipulierfläche (4), das Gewinde (12) in der Öffnung (9), einer Bohrung mit Absatz (17) im Kompressionselement (10) kombiniert mit einem Innensechskant (13) am Ende der Bohrung - über die das Implantat (1) und das Kompressionselement (10) simultan mittels eines Instrumentes (20) manipulierbar sind.

9. Implantat (1) nach einem der Ansprüche 1 - 8, dadurch gekennzeichnet, dass die Fläche der Öffnungen (18a,18b) in der oberen und unteren Begrenzung (5,6) des Rahmens (2) zwischen 40 und 60 %, vorzugsweise 45 und 55 % der in der Mittelebene (8) liegenden gesamten Querschnittfläche des Implantates (1) beträgt.

## Claims

1. Implant (1), in particular for an intervertebral space comprising a frame (2) being through open on the top and the bottom and having a front insertion face (3) and a rear manipulation face (4), whereby
A) the upper (5) and lower boundary (6) of the frame (2) are being shaped convex, so that the implant (1) has an overall lens shape in the mid-plane (8) of the implant (1), the mid-plane (8) including the longitudinal axis (7);
B) the cavity (15) of the implant (1) being through open on the top and the bottom and can be filled with a compressible mass; and
C) the rear manipulation face (4) of the frame (2) is provided with an aperture (9), wherein a compression element (10) is provided, which is unidirectionally displaceable in the aperture (9) in the direction of the longitudinal axis (7) toward the front insertion face (3), whereby
D) by means of the compression element (10) compressible material inserted into the cavity (15) can be forced out on the top and the bottom of the implant.

2. Implant (1) according to claim 1, characterized in that the aperture (9) is provided with an internal thread (12) in order to accept a rotationally symmetrical compression element (10) having a corresponding external thread (11).

3. Implant (1) according to claim 1 or 2, characterized in that the product P = F · T · O⁻¹ of cross sectional area F of the aperture (9), the maximum possible penetration depth T of the compression element (10) into the cavity (15) and the reciprocal of the sum of the areas of the two openings (18a,18b) in the upper and lower boundaries (5;6) of the frame (2) has a value between 0,5 to 1,5 mm.

4. Implant (1) according to claim 1, characterized in that the product P = F · T · O⁻¹ results in a value between 0,6 to 0,9 mm.

5. Implant (1) according to claim 3 or 4, characterized in that the compression element (10) is provided with means (13), preferably in the form of a hexagon socket, wherewith a rotational moment is exertable.

6. Implant (1) according to one of the claims 3 to 5, characterized in that the compression element (10) is provided with means (17), preferably in the form of a bore hole with shoulder, by what means forces in axial direction are exertable.

7. Implant (1) according to one of the claims 1 to 6, characterized in that the rear manipulation face (4) is provided with means (16), preferably a transverse slot, by what means a rotational moment is exertable.

8. Implant (1) according to one of the claims 3 to 5, characterized in that the implant (1) and the compression element (10) are provided with means (16) - preferably in the form of a transverse slot in the rear manipulation face (4), the internal thread (12) in the aperture (9), a bore hole with shoulder (17) within the compression element (10) combined with a hexagonal socket (13) at the end of the bore hole - by what means the implant (1) and the compression element (10) can be simultaneously manipulated by means of an instrument (20).

9. Implant (1) according to one of the claims 1 to 8, characterized in that area of the openings (18a;18b) in the upper and lower boundaries (5;6) of the frame (2) represent between 40 and 60%, preferably between 45 and 55% of the entire cross sectional area in the mid-plane (8) of the implant (2).

## Revendications

1. Implant (1), en particulier pour l'espace intervertébral, constitué d'un cadre (2) ouvert de façon continue dans le haut et dans le bas et doté d'une surface avant d'introduction (3) et d'une surface arrière de manipulation (4),
dans lequel
A) la frontière supérieure (5) et la frontière inférieure (6) du cadre (2) sont de forme convexe, de sorte que l'implant (1) présente globalement une configuration en forme de lentille dans le plan central (8) du cadre (2), qui contient l'axe longitudinal (7) de l'implant (1) ;
B) l'espace intérieur (15) ouvert de manière continue dans le haut et dans le bas de l'implant (1) peut être rempli d'une pâte compressible ; et
C) la surface arrière de manipulation (4) du cadre (2) présente une ouverture (9) dans laquelle est prévu un élément de compression (10) qui peut être déplacé de manière unidirectionnelle dans l'ouverture (9), en direction de l'axe longitudinal (7) suivant la surface avant d'introduction (3), et
E) de la pâte compressible introduite dans l'espace intérieur (15) au moyen de l'élément de compression (10) peut être repoussée hors de l'implant par le haut et par le bas.

2. Implant (1) selon la revendication 1, caractérisé en ce que l'ouverture (9) est dotée d'un filet intérieur (12) pour recevoir un élément de compression (10) à symétrie de rotation qui présente un filet extérieur (11) correspondant.

3. Implant (1) selon la revendication 1 ou 2, caractérisé en ce que le produit P = F.T.O⁻¹ de la surface transversale F de l'ouverture (9), de la profondeur maximale possible de pénétration T de l'élément de compression (10) dans l'espace intérieur (15) et de la réciproque de la somme des surfaces des deux ouvertures (18a, 18b) de la frontière supérieure et de la frontière inférieure (5, 6) du cadre (2) donne une valeur de 0,5 à 1,5 mm.

4. Implant (1) sur la revendication 3, caractérisé en ce que le produit P = F.T.O⁻¹ a une valeur de 0,6 à 0,9 mm.

5. Implant (1) selon la revendication 3 ou 4, caractérisé en ce que l'élément de compression (10) présente des moyens (13), de préférence en forme d'hexagone intérieur, par lequel un couple de rotation peut être exercé.

6. Implant (1) selon l'une des revendications 3 à 5, caractérisé en ce que l'élément de compression (10) présente des moyens (17), de préférence sous la forme d'un alésage à gradin, par l'intermédiaire desquels des forces peuvent être exercées dans la direction axiale.

7. Implant (1) selon l'une des revendications 1 à 6, caractérisé en ce que la surface arrière de manipulation (4) présente des moyens (16), de préférence une fente transversale, par l'intermédiaire desquels un couple de rotation peut être exercé.

8. Implant (1) selon l'une des revendications 3 à 5, caractérisé en ce que l'implant (1) et l'élément de compression (10) présentent des moyens (16), de préférence sous la forme d'une fente transversale dans la surface arrière de manipulation (4) d'un filet (12), dans l'ouverture (9), d'un alésage à gradin (17) dans l'élément de compression (10), combiné avec un hexagone intérieur (13) à l'extrémité de l'alésage, à l'aide desquels l'implant (1) et l'élément de compression (10) peuvent être manipulés simultanément au moyen d'un instrument (20).

9. Implant (1) selon l'une des revendications 1 à 8, caractérisé en ce que la surface des ouvertures (18a, 18b) dans la frontière supérieure et de la frontière inférieure (5, 6) du cadre (2) vaut entre 40 et 60 %, de préférence entre 45 et 55 % de la surface transversale totale, située dans le plan central (8), de l'implant (1).
